# EUROPÄISCHE PATENTSCHRIFT

(11) **EP 2 040 072 B1**
(45) Veröffentlichungstag und Bekanntmachung des Hinweises auf die Patenterteilung: **02.01.2013**
(21) Anmeldenummer: 07018674.7
(22) Anmeldetag: 22.09.2007
(51) Int. Cl.: G01N 21/78, G01N 27/327

(54) **Analysesystem zur Bestimmung der Konzentration eines Analyten in einer Körperflüssigkeit**
Analysis system for measuring the concentration of an analyte in a bodily fluid
Système d'analyse destiné à la détermination de la concentration d'un analyte dans un liquide corporel

(43) Veröffentlichungstag der Anmeldung: 25.03.2009
(73) Patentinhaber: Roche Diagnostics GmbH, 68305 Mannheim (DE); F.Hoffmann-La Roche AG, 4070 Basel (CH)
(72) Erfinder: Haar, Hans-Peter, 69168 Wiesloch (DE)
(74) Vertreter: Petirsch, Markus

(56) Entgegenhaltungen:
- EP-A- 1 702 561
- WO-A-01/67099
- GB-A- 2 024 432
- US-A- 5 676 143
- US-A1- 2002 016 535
- US-A1- 2003 153 820
- US-A1- 2005 023 137
- US-A1- 2007 173 761
- ALFORD ET AL: "Bioprocess control: Advances and challenges", COMPUTERS & CHEMICAL ENGINEERING, PERGAMON PRESS, OXFORD, GB LNKD- DOI:10.1016/J.COMPCHEMENG.2006.05.039, vol. 30, no. 10-12, 12 September 2006 (2006-09-12), pages 1464-1475, XP025089007, ISSN: 0098-1354 [retrieved on 2006-09-12]

## Beschreibung

Die vorliegende Erfindung betrifft ein Analysesystem zur Bestimmung eines Analyten in einer Körperflüssigkeit, umfassend ein Testelement mit einem Reagenzsystem, dessen Reaktion mit dem Analyten zu einer für das gewünschte analytische Resultat charakteristischen nachweisbaren Änderung führt, und ein Auswertegerät mit einer Mess- und Auswerteeinheit.

Im Stand der Technik sind Analysesysteme zur Bestimmung eines Analyten in einer Körperflüssigkeit bekannt, die disposible Testelemente, Testträger oder Teststreifen verwenden. Derartige Systeme werden zur Bestimmung der Konzentration verschiedener Analyten eingesetzt. Beispielsweise wird der Glucosegehalt oder der Cholesteringehalt im Blut ermittelt.

Die Testelemente enthalten in der Regel ein Reagenzsystem aus einem oder mehreren Reagenzien, deren Reaktion mit der Probenflüssigkeit zu einer nachweisbaren Veränderung führt, die mit dem Analysesystem gemessen werden kann. Bei photometrisch arbeitenden Analysesystemen findet in dem Testelement eine Farbänderung in einer Nachweisschicht als Reaktion des Testelements mit der Probe statt, die von einer zu dem Analysesystem gehörenden Mess- und Auswerteeinheit photometrisch gemessen wird. Beispielsweise wird die Intensität des vom Testelement reflektierten Lichtes untersucht.

Alternativ werden auch sogenannte elektrochemische Analysesysteme verwendet, bei denen die Aufgabe einer Flüssigkeitsprobe auf das Testelement zu einer elektrochemischen Reaktion führt, die als nachweisbare Ladungsänderung, Stromfluss oder Spannungsänderung detektiert wird.

Die bekannten Analysesysteme arbeiten bei sachgemäßer Anwendung in der Regel zuverlässig. Dennoch kann es aufgrund von Applikationsfehlern beim Analysegerät oder beim Testelement zu fehlerhaften Messergebnissen kommen. Obwohl große Sorgfalt bei der Herstellung solcher Analysesysteme und Testelement verwendet wird, ist eine hohe Qualitätskontrolle gewünscht, bei der Fehlfunktionen des Messgerätes ermittelt werden, um fehlerhafte Ergebnisse zu vermeiden.

Insbesondere bei Glucosemesssystemen, bei denen der Glucosegehalt im Blut ermittelt wird, wird eine hohe Qualität und eine geringe Fehlertoleranz gefordert. Die Ergebnisse der Konzentrationsermittlung des Glucosegehalts im Blut sind Grundlage für die Therapie eines Patienten. Die Dosierung des Insulins wird aufgrund des ermittelten Glucosegehalts bestimmt, so dass ein fehlerfreier Analysewert besonders wichtig ist. Eine Therapierung von Patienten, die auf einem fehlerhaften Messergebnis basieren und deshalb zu einer falschen Zugabe von Insulin (zu geringe oder zu hohe Insulindosis) führen, kann zu körper- und lebensbedrohlichen Situationen führen.

Aus diesem Grund werden redundante Systeme verwendet, bei denen beispielsweise zwei parallel geschaltete photometrische Detektoren angeordnet sind, die das von einer Analysezone remittierte Licht ermitteln. Ein derartiges System ist in der US 6,955,060 beschrieben. Alternativ können zwei separierte Analysezonen mit zwei Lichtquellen beleuchtet werden, um mit einem Detektor zwei unterschiedliche Analyseergebnisse zu bestimmen.

Fehlereinflüsse von Analysesystemen können allgemein auf mechanischer, messtechnischer oder auf elektronischer Ebene wirken. Die digitalen Subsysteme selbst lassen sich zwar gut kontrollieren, bei digitalanalogen Teilsystemen ist eine Qualitätskontrolle zur Sicherstellung einer Messgenauigkeit bzw. Fehlerfreiheit deutlich schwieriger.

Um die Messgenauigkeit zu erhöhen und insbesondere Fehler zu vermeiden, könnten von einem Patienten mehrere voneinander unabhängige Messungen durchgeführt werden. Beispielsweise könnte ein Anwender zwei unabhängige Messungen nacheinander mit dem gleichen Gerät aber mit zwei verschiedenen Testelementen durchführen. Ein derartiges Vorgehen ist für den Anwender jedoch im praktischen Gebrauch nicht zumutbar.

Alternativ könnte, um die Messsicherheit zu erhöhen, eine Messung mit zwei völlig voneinander unabhängigen Geräten stattfinden. Auch dies führt zu einem deutlich erhöhten Mess- und Kostenaufwand, der nicht praktikabel ist.

Es ist somit Aufgabe der vorliegenden Erfindung, die bekannten Analysesysteme dahingehend zu verbessern, dass die Zuverlässigkeit erhöht wird.

Gelöst wird die vorliegende Aufgabe durch ein Analysesystem mit den Merkmalen des Anspruchs 1. Die in den Unteransprüchen definierten Weiterbildungen des erfindungsgemäßen Analysesystems definieren zusätzliche vorteilhafte, nicht selbstverständliche Merkmale.

Das erfindungsgemäße Analysesystem zur Bestimmung eines Analyten in einer Körperflüssigkeit umfasst ein Testelement mit einem Reagenzsystem, dessen Reaktion mit dem Analyten zu einer nachweisbaren Änderung führt, die für das gewünschte analytische Resultat charakteristisch ist. Das Testelement hat eine Probenaufgabezone und zwei Analysezonen. Das Analysesystem umfasst ein Analysegerät mit einer Mess-und Auswerteeinheit. Sie beinhaltet (mindestens) zwei Analog-Messeinheiten, in denen ein in der Änderung in jeweils einer der Analysezonen korrespondierendes analoges Messsignal erzeugt wird, zwei Analog-Digital-Wandler zum Digitalisieren des analogen Messsignals, eine Vergleichereinheit zum Vergleichen von Kontroll-Datenwerten, die auf den digitalisierten Messsignalen basieren, und eine Verarbeitungsendeinheit. Wenn die ermittelte Abweichung zwischen den verglichenen Kontroll-Datenwerten der digitalisierten Messsignale kleiner als ein vordefinierter Wert ist, erfolgt in der Verarbeitungsendeinheit die Freigabe mindestens eines der Kontroll-Datenwerte und die Weiterverarbeitung des bzw. der freigegebenen Kontroll-Datenwerte zu dem gesuchten analytischen Resultat.

Im Sinne der vorliegenden Erfindung ist der Begriff "korrespondierendes" analoges Messsignal derart zu verstehen, dass der Wert des Messsignals ein Maß für die Änderung in der Analysezone ist. Das Messsignal kann proportional zu der Änderung sein, was nicht im streng mathematischen Sinne zu verstehen ist, sondern auch die üblicherweise nicht linearen Zusammenhänge zwischen einer Analytkonzentration und einem Messsignal umfasst.

Im Rahmen der Erfindung wurde festgestellt, dass es besonders wichtig ist, die gesamten Auswertekanäle, die die Analysezonen, die Analog-Messeinheit und den Analog-Digital-Wandler umfassen, redundant aufzubauen, da Fehlereinflüsse sowohl auf mechanischer oder messtechnischer Ebene, als auch auf elektronischer Ebene, beispielsweise in Form des sogenannten Bit-Flips, auftreten können. Während im Stand der Technik Ausführungen bekannt sind, die die digitalen Komponenten eines Analysesystems kontrollieren, beispielsweise durch Überprüfung durch Software-Algorithmen, stößt die Kontrolle von kombinierten Digital-Analog-Systemen oder Teilsystemen auf große Schwierigkeiten. Dies wird durch die redundante Anordnung der Messkanäle gelöst. Alternativ können nicht nur zwei Auswertekanäle, sondern auch mehr als zwei redundante Auswertekanäle implementiert werden.

Erfindungsgemäß ist das Analysesystem derart redundant aufgebaut, dass zwei unabhängige Messungen parallel zueinander durchgeführt werden. Jeder der beiden Analysezonen ist je eine Analog-Messeinheit und je ein Analog-Digital-Wandler zugeordnet, so dass in der Analog-Messeinheit ein analoges Messsignal erzeugt wird, das der Änderung in der Analysezone proportional ist bzw. das ein Maß für die Änderung ist. Eine den Analog-Digital-Wandler umfassende Digitaleinheit digitalisiert das Messsignal aus der Analog-Messeinheit. Das Analysesystem umfasst somit zwei unabhängig voneinander arbeitende Auswertekanäle, die nicht nur die analoge Messeinheit, sondern auch die als Analog-Digital-Wandler ausgebildete Digitaleinheit umfassen. Beide Messungen sind somit völlig unabhängig voneinander und können parallel und gleichzeitig durchgeführt werden. Das Analysesystem liefert sehr sichere und zuverlässige Ergebnisse. Da der Benutzer nichts von den parallelen Messungen merkt, ist die Bedienerfreundlichkeit des Systems sehr hoch. Messfehler, die in einem der Auswertekanäle auftreten können, werden sofort erkannt. Derartige Analysesysteme sind insbesondere für die Glucoseermittlung geeignet, da eine sehr geringe Fehlertoleranz, eine sehr hohe Qualitätskontrolle und gesicherte Analyseergebnisse gefordert werden.

Eine Voraussetzung hierfür ist die Verwendung von zwei verschiedenen Analysezonen. Der Begriff Analysezone ist dabei so zu verstehen, dass er nicht nur einen Bereich, sondern auch ein Analysevolumen bzw. ein Auswertevolumen umfassen kann. Die beiden Analysezonen müssen also räumlich nicht übereinstimmende Volumen sein. Überlappungen der beiden Analysezonen bzw. ein Teilvolumen oder eine Teilmenge, die beiden Zonen gemeinsam ist, ist durchaus möglich. Die beiden Analysezonen dürfen jedoch nicht identisch sein, da andernfalls die Qualität des gesicherten Messergebnisses reduziert würde.

Das erfindungsgemäße Analysesystem kann zur weiteren Erhöhung der Messgenauigkeit und der Vermeidung von Fehlmessungen auch mit zusätzlichen Maßnahmen wie der Eigenüberwachung der Analysezonen, beispielsweise durch Benetzungskontrolle oder Impedanzmessungen, kombiniert werden. Allerdings werden derartige Fehler ebenfalls von dem erfindungsgemäßen Analysesystem erkannt.

Das Analysesystem weist den weiteren Vorteil auf, dass es in anderen Geräten integriert werden kann. Beispielsweise kann das erfindungs-gemäße Analysesystem in ein kombiniertes Stech- und Analysesystem integriert werden, bei dem eine Wunde in einem Körperteil erzeugt wird und Blut bzw. eine Körperflüssigkeit aus dieser Wunde aufgenommen und in das System transportiert wird. Die Körperflüssigkeit muss von dem Stechsystem lediglich zu den Analysezonen des Analysesystems transportiert werden, in denen eine messbare Änderung durch die Reaktion mit der Körperflüssigkeit hervorgerufen wird.

Erfindungsgemäß werden in der Vergleichereinheit digitale Kontroll-Datenwerte miteinander verglichen, die jeweils auf den digitalisierten Messsignalen basieren. Diese Kontroll-Datenwerte repräsentieren die Messsignale, die aufgrund der detektierten Änderung in den Analysezonen ermittelt wurden. Ein Kontroll-Datenwert ist jedenfalls ein "Verarbeitungswert" oder "Zwischenwert", der aus dem digitalisierten Messwert des Messsignals ermittelt (z. B. berechnet) wird. Diese Zwischenwerte können auch Rohdaten ("raw data") sein. In diesem Fall sind sie mit.den digitalisierten Messsignalen identisch. Somit ist der Kontroll-Datenwert ein in der Verarbeitungskette zwischen der Digitalisierung des analogen Messsignals und dem gesuchten analytischen Resultat erzeugter Wert. Der Kontroll-Datenwert kann bevorzugt auch ein ermittelter Konzentrationswert des Analyten sein.

Das analytische Resultat ist der Wert, der durch Weiterverarbeitung wenigstens eines Kontroll-Datenwertes erzeugt wird. Es wird beispielsweise an den Benutzer ausgegeben oder an ein weiteres Gerät übertragen. Das analytische Resultat kann alternativ auf zwei Konzentrationswerten beruhen, die aus den digitalisierten Messsignalen ermittelt werden. Die Konzentrationswerte können auch auf den Kontroll-Datenwerten basieren, die ihrerseits aus den digitalisierten Messsignalen erzeugt werden.

Bevor eine Weiterverarbeitung der Kontroll-Datenwerte zu dem gesuchten analytischen Resultat erfolgt, muss wenigstens einer der Kontroll-Datenwerte von der Verarbeitungsendeinheit freigegeben werden. Dies geschieht dann, wenn die in der Vergleichereinheit ermittelte Abweichung zwischen den Kontroll-Datenwerten kleiner als eine vordefinierte Grenze ist, die als Threshold-Wert bezeichnet wird.

Nur wenn die beiden Auswertekanäle gleiche (oder identische) Kontroll-Datenwerte liefern oder deren Abweichung kleiner als die vorgeschriebene Grenze (Toleranzwert) ist, sind beide Messungen in den Auswertekanälen fehlerfrei. Sobald ein Fehler in einem der Auswertekanäle auftritt, sei es durch einen Fehler in der Analog-Messeinheit, im Analog-Digital-Wandler oder beispielsweise durch eine unvollständige Benetzung einer der Analysezonen, wird dieser Fehler aufgrund einer zu großen Abweichung der beiden Kontroll-Datenwerte umgehend erkannt. Die Verarbeitung zu einem analytischen Resultat wird dann abgebrochen oder wiederholt. Somit wird sichergestellt, dass kein fehlerhaftes Ergebnis ausgegeben wird. Zusätzlich kann ein Fehlersignal erzeugt und ausgegeben werden.

Das erfindungsgemäße Analysesystem eignet sich sowohl für die optische Erfassung der in dem Reagenzsystem hervorgerufenen Änderung, beispielsweise einer Farbveränderung, wie auch für eine elektrochemische Auswertung, indem ein Stromfluss bzw. eine Spannungsänderung detektiert wird. Die optischen Messungen können hierbei Fluoreszenzmessungen, Lumineszenzmessungen oder ähnliches umfassen. Gebräuchlich sind Messungen eines durch diffuse Reflexion erzeugten Lichtsignals oder Messungen, die auf dem Prinzip der Transmission beruhen.

Das auf einer optischen Erfassung beruhende Analysesystem kann in einer einfachen Ausführungsform eine Mess- und Auswerteeinheit umfassen, in der ein konstantes Lichtsignal abgestrahlt wird und in der Analog-Messeinheit eine Änderung des empfangenen Messsignals gemessen wird. In einer aufwendigeren Ausführung werden modulierte oder gepulste Lichtsignale erzeugt. Die Analog-Messeinheit ist derart ausgebildet, dass das empfangene Messsignal entsprechend zeitlich zugeordnet wird, um z.B. Signale aus dem "Untergrund" (Noise) zu erhalten.

Bei der Verwendung elektrochemischer Analysesysteme (mit einer elektrochemischen Erfassung der Änderung) kann in einer einfachen Ausführungsform der Mess- und Auswerteeinheit eine Polarisationsspannung an die Analysezonen angelegt werden und in der Analog-Messeinheit ein durch die chemische Umsetzung hervorgerufener Strom gemessen werden. Es können allerdings auch in zeitlicher Reihenfolge unterschiedliche Wechselspannungen angelegt werden, um zusätzlich zur Strommessung aus der Bestimmung der Impedanz weitere Informationen zu gewinnen. Die Gewinnung zusätzlicher Informationen ist beispielsweise in der US 2006/0156796 beschrieben.

Der im Rahmen dieser Erfindung verwendete (summarische) Begriff "analoges Messsignal" bezieht sich folglich auf den Teil des Analysesystems, bei dem analoge Signale erzeugt und auch vermessen werden. Die analogen Messsignale können je nach Ausführungsform des Analysesystems folglich auch komplexe zeitliche Verläufe umfassen.

Unabhängig von der Art der Messung (optische Messung oder elektrochemische Messung) können die beiden Auswertekanäle entweder gleich sein oder zumindest teilweise symmetrisch aufgebaut sein. Bei einer optischen Messung wird bevorzugt nur ein optischer Sender zur Emission von Licht auf die (beiden) Analysezonen verwendet. Die Mess-und Auswerteeinheit umfasst bevorzugt zwei optische Empfänger, von denen jeder einer Analysezone derart zugeordnet ist, dass der optische Empfänger das von der Analysezone reflektierte Licht empfängt. Die optischen Empfänger können beispielsweise Photozellen oder Photodioden oder ähnliche Empfänger sein. In einer bevorzugten Ausführungsform erzeugt jede der beiden Analog-Messeinheiten ein analoges Messsignal, das mit dem empfangenen reflektierten Licht des jeweiligen optischen Empfängers proportional ist, das von der jeweiligen Analysezohe reflektiert wird.

Sind die beiden Messkanäle identisch aufgebaut, so umfasst das Analysegerät zwei optische Sender, wobei je ein Sender Licht auf eine der beiden Analysezonen aussendet. Die beiden optischen Sender werden vorteilhafterweise von einem Signalgenerator angesteuert oder gespeist.

In einer bevorzugten Ausführungsform sind die Daten-Kontrollwerte die gesuchten Konzentrationswerte. Der Vergleichereinheit werden dann zwei Konzentrationswerte zum Vergleichen zugeführt. Der vordefinierte Wert als zulässige Grenze für die Abweichung der Konzentrationswerte ist ein Referenzkonzentrationswert. In dieser bevorzugten Ausführungsform wird aus dem digitalisierten Messsignal bereits ein gesuchter Konzentrationswert erzeugt, der dann in der Vergleichereinheit und die Verarbeitungsendeinheit weiterverarbeitet wird, wenn die Abweichung der beiden Konzentrationswerte kleiner als der Referenzkonzentrationswert ist.

Die Erfindung wird nachfolgend anhand von in den Figuren dargestellten bevorzugten Ausführungsformen näher erläutert. Die dort dargestellten Besonderheiten können einzeln oder in Kombination verwendet werden, um bevorzugte Ausgestaltungen der Erfindung zu schaffen. Es zeigen:
- Figur 1: ein schematisches Blockschaltbild eines erfindungsgemäßen Analysesystems;
- Figur 2: ein schematisches Blockschaltbild einer weiteren Ausführungsform eines Analysesystems;
- Figur 3: das Blockschaltbild eines auf einer optischen Messung beruhenden Analysesystems nach Figur 2;
- Figur 4: ein Blockschaltbild eines auf einer elektrochemischen Messung beruhenden Analysesystems nach Figur 2;
- Figur 5: ein Prinzipschaltbild eines Analysegerätes und eines in Form eines Sticks ausgeführten Testelements.

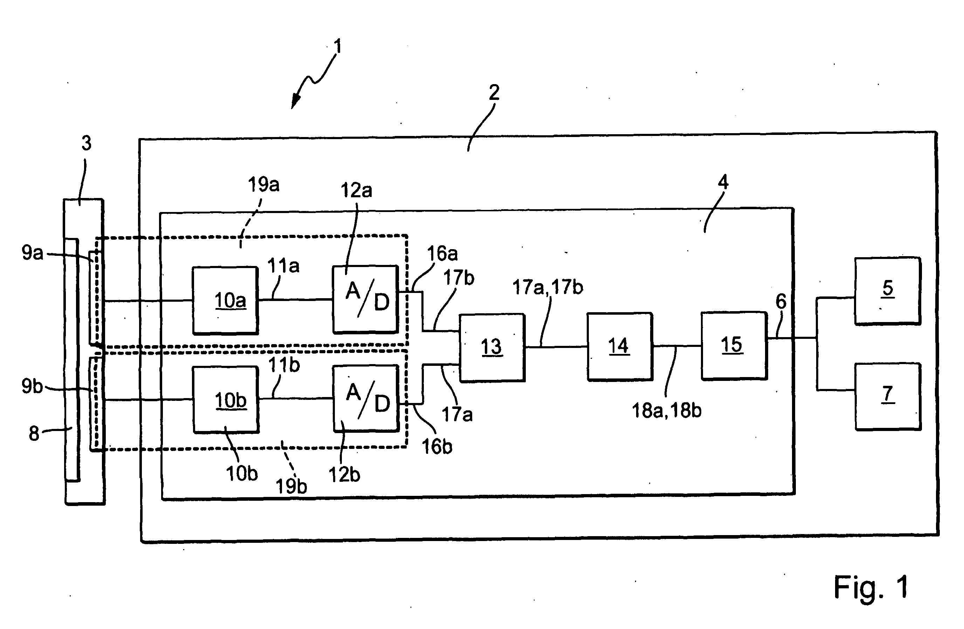

Die Figuren 1 bis 4 zeigen ein erfindungsgemäßes Analysesystem 1 mit einem Analysegerät 2 und einem Testelement 3. Das Analysegerät 2 weist eine Mess- und Auswerteeinheit 4 auf und bevorzugt eine Ausgabeeinheit 5, an der das von der Mess- und Auswerteeinheit 4 ermittelte analytische Resultat 6 oder ein Fehlersignal ausgegeben wird.

Die Ausgabeeinheit 5 kann das analytische Resultat 6 als optisches Signal, beispielsweise als Zahlenwert, z.B. ein Glucosewert, oder in Form von verschiedenen Symbolen anzeigen. Daneben ist auch eine akustische Ausgabe der Resultate möglich. Die Ausgabeeinheit 5 kann neben dem analytischen Resultat 6 weitere Informationen liefern, beispielsweise kann eine erfolgreiche Messung im Klartext oder als Signalton ausgegeben werden, ebenso ein Fehlersignal. Darüber hinaus können zusätzliche Informationen für den Benutzer angezeigt werden.

Optional umfasst das Analysegerät 2, wie in den Figuren 1 bis 4 gezeigt, einen Speicher 7, in dem Messergebnisse gespeichert werden, beispielsweise Zwischenwerte oder analytische Resultate 6. Auf diese Weise kann eine Historie der Messergebnisse angelegt werden. Der Speicher kann beispielsweise über eine Schnittstelle von einem angeschlossenen Gerät oder einem Computer ausgelesen werden. Die analytischen Resultate 6 oder weitere Informationen können alternativ oder zusätzlich zur Anzeige an der Ausgabeeinheit 5 auch über die Schnittstelle an weitere Geräte übermittelt werden, um weiterverarbeitet oder archiviert zu werden.

Das Testelement 3 hat eine Probenaufgabezone 8 und zwei Analysezonen 9a,9b. Bevorzugt ist die Probenaufgabezone 8 an einer Oberseite des Testelements 3 angeordnet, die Analysezonen 9a,9b sind an einer Unterseite des Testelements positioniert. In einer bevorzugten Ausführungsform weist das Testelement 3 zwischen der Probenaufgabezone 8 und den beiden Analysezonen 9a, 9b einen Kapillarkanal auf, mittels dem eine Körperflüssigkeit, die auf die Probenaufgabezone 8 übertragen wird, an die Analysezonen 9a,9b geleitet wird. Der Kapillarkanal ist in den Figuren 1 bis 4 nicht dargestellt.

Sowohl die Probenaufgabezone 8 als auch die Analysezonen 9a, 9b und der Kapillarkanal können Teil des Reagenzsystems des Testelements 3 sein, indem bei einer Reaktion mit einer Körperflüssigkeit mit einem Anatyten eine für den Analyten charakteristische nachweisbare Änderung hervorgerufen wird, die an den Analysezonen 9a, 9b detektiert werden kann. Wie aus den Figuren ersichtbar ist, sind die beiden Analysezonen 9a, 9b voneinander verschieden, wobei ein überlappender Bereich möglich ist. Beide Analysezonen 9a, 9b haben folglich ein unterschiedliches Analysevolumen.

Die in den Analysezonen 9a, 9b detektierbare Änderung des Reagenzsystems des Testelements 3 wird von zwei Analog-Messeinheiten 10a, 10b erfasst, in denen je ein analoges Messsignal 11a bzw. 11b erzeugt wird, das der Änderung in der jeweiligen Analysezone 9a bzw. 9b proportional ist.

Die Mess- und Auswerteeinheit 4 umfasst zwei Analog-Digital-Wandler 12a, 12b (A/D-Wandler), deren Eingang mit dem Ausgang der jeweiligen Analog-Messeinheit 10a, 10b verbunden ist und an deren Eingang das analoge Messsignal 11a bzw. 11b anliegt.

In dem Ausführungsbeispiel nach Figur 1 umfasst die Mess- und Auswerteeinheit 3 eine Vergleichereinheit 13, eine Auswerteeinheit 14 und eine Verarbeitungsendeinheit 15, die in der Verarbeitungskette hintereinander geschaltet sind.

Die am Eingang der A/D-Wandler 12a, 12b anliegenden analogen Signale 11a bzw. 11b werden digitalisiert und von den A/D-Wandlern 12a, 12b als digitalisierte Messsignale 16a, 16b an die Vergleichereinheit 13 in Form von digitalen Kontroll-Datenwerten 17a, 17b übertragen. Bei der Ausführungsform nach Figur 1 sind die Kontroll-Datenwerten 17a,

17b mit den digitalisierten Messsignalen 16a, 16b identisch. Die Kontroll-Datenwerte 17a, 17b werden in der Vergleichereinheit 13 miteinander verglichen und an die Auswerteeinheit 14 übermittelt. Aus den Kontroll-Datenwerten 17a, 17b werden in der Auswerteeinheit 14 die gesuchten Konzentrationswerte 18a, 18b mittels eines Auswertealgorithmus ermittelt. Der Auswertealgorithmus umfasst eine Zuordnung zwischen Kontroll-Datenwerten 17a, 17b und Konzentrationswerten 18a, 18b. Diese Zuordnung kann beispielsweise in Form einer Tabelle in der Auswerteeinheit 14 hinterlegt sein.

Wenn die in der Vergleichereinheit 13 ermittelte Abweichung zwischen den Kontroll-Datenwerten 17a, 17b kleiner als ein vordefinierter Wert (Schwellwert) ist, erfolgt in der Verarbeitungsendeinheit 15 die Freigabe wenigstens eines der Kontroll-Datenwerte 17a, 17b bzw. der auf den Kontroll-Datenwerten 17a, 17b beruhenden Konzentrationswerte 18a, 18b. Die freigegebenen Kontroll-Datenwerte 17a, 17b bzw. Konzentrationswerte 18a, 18b werden in der Verarbeitungsendeinheit 15 zu dem analytischen Resultat 6 weiterverarbeitet, das am Ausgang der Mess-und Auswerteeinheit 4 zur Weiterverarbeitung zur Verfügung steht.

Vorzugsweise werden von der Verarbeitungsendeinheit 15 beide Kontroll-Datenwerte 17a, 17b freigegeben, wenn die Abweichung zwischen den beiden Kontroll-Datenwerten 17a, 17b kleiner als der vordefinierte Wert ist. Bevorzugt erfolgt dann deren Weiterverarbeitung zu dem gewünschten analytischen Resultat 6 auf Basis beider Kontroll-Datenwerte 17a, 17b. Besonders bevorzugt wird das analytische Resultat 6 aus dem Mittelwert der beiden Kontroll-Datenwerte 17a, 17b gebildet. In der Verarbeitungsendeinheit 15 kann das arithmetische Mittel oder das geometrische Mittel aus den beiden Werten gebildet werden. Andere Mittelwertbildungen, beispielsweise ein gewichteter Mittelwert, sind ebenfalls möglich. Bevorzugt kann das analytische Resultat 6 auch der Mittelwert der beiden Konzentrationswerte 18a, 18b sein.

Alternativ kann in der Verarbeitungsendeinheit 15 das analytische Resultat 6 entweder aus dem kleineren oder aus dem größeren der beiden Kontroll-Datenwerte 17a, 17b ermittelt werden. Anstelle der Kontroll-Datenwerte 17a, 17b können auch hier die Konzentrationswerte 18a, 18b Verwendung finden.

Ist die ermittelte Abweichung zwischen den Kontroll-Datenwerten 17a, 17b größer als der vordefinierte Wert, erfolgt bevorzugt keine Freigabe in der Verarbeitungsendeinheit 15. Statt dessen wird ein Fehlersignal erzeugt, das an die Ausgabeeinheit 5 übermittelt wird.

In der Ausführungsform nach Figur 1 weist das Analysesystem 1 zwei redundant aufgebaute Auswertekanäle 19a, 19b auf, die jeweils eine Analysezone 9a, 9b, eine Analog-Messeinheit 10a, 10b und einen A/D-Wandler 12a, 12b umfassen.

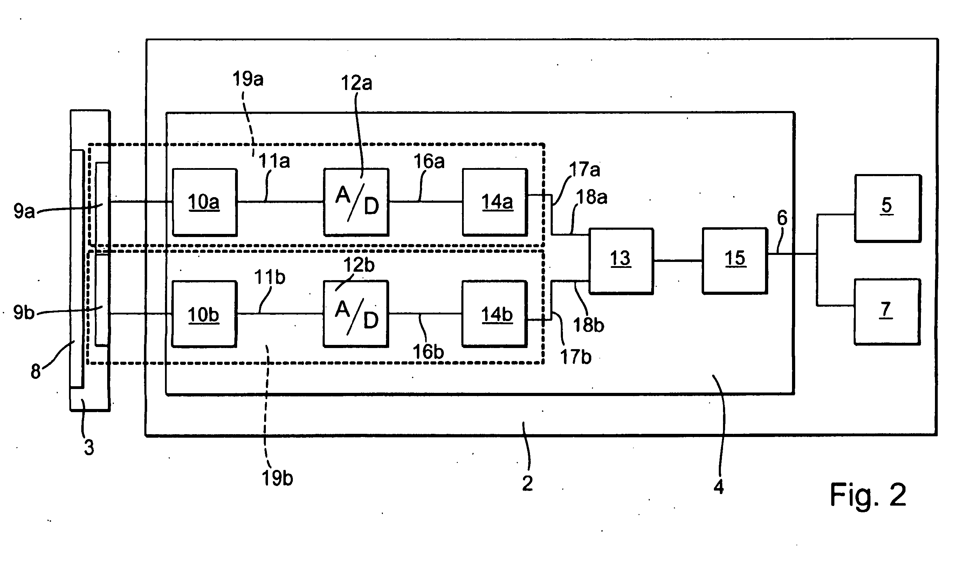

Die Ausführungsform nach Figur 2 hat ebenfalls zwei redundante Auswertekanäle 19a, 19b. Diese Auswertekanäle 19a, 19b umfassen jeweils neben der Analysezone 9a, 9b, der Analog-Messeinheit 10a, 10b und den A/D-Wandler 12, 12b zusätzlich eine Auswerteeinheit 14a, 14b. Die Mess- und Auswerteeinheit 4 weist bevorzugt also zwei Auswerteeinheiten 14a, 14b auf. In jeder der Auswerteeinheiten 14a, 14b wird aus dem digitalisierten Messsignal 16a, 16b mittels eines Auswertealgorithmus ein Kontroll-Datenwert 17a, 17b erzeugt. Der Auswertealgorithmus umfasst eine Zuordnung zwischen Werten des digitalisierten Messsignals 16a, 16b und den Kontroll-Datenwerten 17a, 17b.

Bevorzugt sind die Kontroll-Datenwerte 17a, 17b die gesuchten Konzentrationswerte 18a, 18b. Der Auswertealgorithmus umfasst in diesem bevorzugten Fall eine Zuordnung zwischen Werten des digitalisierten Messsignals 16a, 16b und Konzentrationswerten 18a, 18b, so dass in den Auswerteeinheiten 14a, 14b Konzentrationswerte 18a, 18b ausgegeben werden, die der detektierten Änderung in den Analysezonen 9a, 9b entsprechen. In der Vergleichereinheit 13 werden zwei Konzentrationswerte 18a, 18b miteinander verglichen.

Bevorzugt wird die Zuordnung des Auswertealgorithmus der Auswerteeinheiten 14, 14a, 14b durch eine Kalibration ermittelt. Während der Kalibration werden zu Referenzkonzentrationswerten digitale Referenzmesssignale erzeugt und dann beispielsweise in Form einer Tabelle oder ähnlichem in einem Speicher, beispielsweise einem RAM, eine EPROM, einem EEPROM oder dergleichen hinterlegt. Bevorzugt wird ein nichtflüchtiger Speicher verwendet, der beispielsweise in einer der Digitalkomponenten, beispielsweise der Auswerteeinheit 14, 14a, 14b, integriert sein kann.

Durch die Ermittlung der Konzentrationswerte durch Kalibration im vorhinein kann zum einen eine sehr einfache und schnelle Umwandlung der digitalisierten Messwerte in Konzentrationswerte erfolgen. Zum anderen können auch chargenspezifische Zuordnungen in den Geräten hinterlegt werden, so dass abhängig von den verwendeten Testelementen (beispielsweise Teststreifen oder ähnliches) unterschiedliche Zuordnungen zwischen den detektierten Änderungen der Analysezonen der Testelemente und den Konzentrationswerten realisiert werden können.

Da die digitale Auswerteeinheit 14a, 14b redundant ausgebildet ist, werden Fehler (beispielsweise sporadische Fehler, Bitflips) in der Auswerteeinheit 14a, 14b erkannt, die unter bestimmten Betriebsbedingungen oder Umgebungsbedingungen auftreten und nicht systematisch erkennbar sind. Die Zuverlässigkeit des erfindungsgemäßen Analysesystems 1 wird durch das Aufdecken derartiger Fehler nochmals erhöht.

Aus den miteinander verglichenen Konzentrationswerten 18a, 18b wird in der Verarbeitungsendeinheit 15 das analytische Resultat 6 ermittelt, das wie vorstehend erläutert nach Freigabe durch die Verarbeitungsendeinheit 15 aus beiden oder einem der Konzentrationswerte 18a, 18b ermittelt wird.

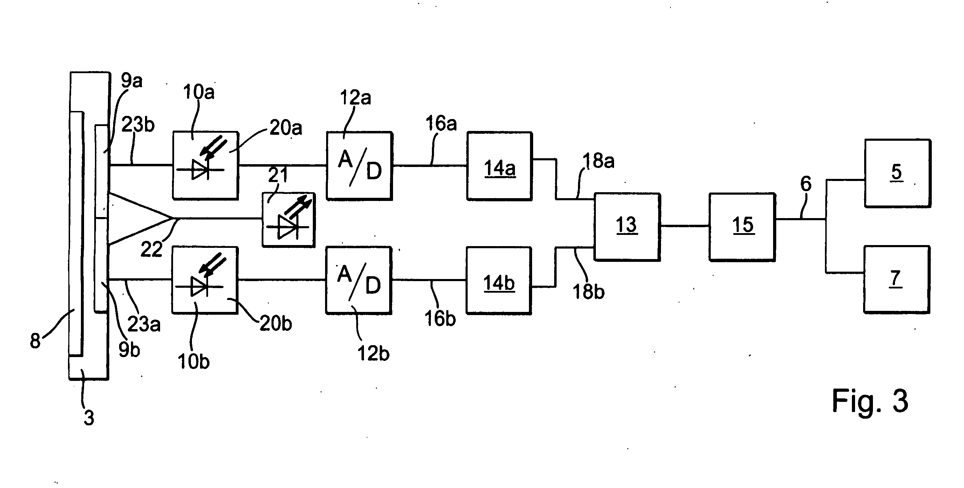

Figur 3 zeigt eine Ausführungsform des erfindungsgemäßen Analysesystems 1, bei der die beiden Auswertekanäle 19a, 19b auch die (redundanten) Auswerteeinheiten 14a, 14b umfassen. In dieser bevorzugten Ausführungsform weist die Mess- und Auswerteeinheit 4 jeweils zwei optische Empfänger 20a, 20b zum Empfang von reflektiertem Licht von je einer der Analysezonen 9a, 9b auf. Der optische Empfänger 20a empfängt reflektiertes Licht von der Analysezone 9a; der optische Empfänger 20b empfängt reflektiertes Licht von der Analysezone 9b. Die optischen Empfänger 20a, 20b sind in die Analog-Messeinheiten 10a, 10b integriert. Das Analysegerät 2 hat einen optischen Sender 21 zur Emission von Licht auf die beiden Analysezonen 9a, 9b des Testelements 3. Das emittierte Licht wird an der Analysezone 9a, 9b reflektiert und von den beiden Analog-Messeinheiten 10a, 10b, die die optischen Empfänger 20a bzw. 20b beinhalten, empfangen, um je ein analoges Messsignal 11a, 11b zu erzeugen, das dem reflektierten Licht der entsprechenden Analysezone 9a, 9b proportional ist.

Besonders bevorzugt ist, wie in Figur 3 gezeigt, ein Lichtleiter 22 zwischen dem optischen Sender 21 und den Analysezonen 9a, 9b angeordnet, mittels dem wenigstens eine der Analysezonen 9a, 9b mit Licht bestrahlt wird. Besonders bevorzugt werden beide Analysezonen 9a, 9b mit Licht beleuchtet, so dass eine Reflexion des Lichtes an der jeweiligen Analysezone 9a, 9b auftritt. Bevorzugt ist zwischen den optischen Empfängern 20a, 20b und der zugeordneten Analysezone 9a, 9b je ein Lichtleiter 23a, 23b angeordnet, um das von der entsprechenden Analysezone 9a, 9b reflektierte Licht an den optischen Empfänger 20a, 20b zu übertragen.

Die aus den optischen Lichtsignalen erzeugten analogen Messsignale 11a, 11b werden dann in der bekannten Weise wie in der Ausführungsform nach Figur 2 weiterverarbeitet, bis ein analytisches Resultat 6 ermittelt worden ist. Bei dieser Ausführungsform wird, wie bei den anderen Ausführungsformen, eine Freigabe der Kontroll-Datenwerte 17a, 17b bzw. der Konzentrationswerte 18a, 18b bevorzugt nur dann ausgeführt, wenn die ermittelte Abweichung zwischen den Kontroll-Datenwerten 17a, 17b bzw. den Konzentrationswerten 18a, 18b unterhalb einer vordefinierten Grenze liegt. Andernfalls wird die Freigabe verhindert und ein Fehlersignal erzeugt, das an der Ausgabeeinheit 5 ausgegeben werden kann.

In einer alternativen Ausführungsform eines Analysesystems 1, das auf einer optischen Messung basiert, können auch zwei optische Sender vorgesehen sein, von denen jeder eine Analysezone 9a, 9b mit Licht bestrahlt. Die beiden Auswertekanäle 19a, 19b umfassen dann jeweils einen der beiden optischen Sender, so dass beide Kanäle identisch aufgebaut sind.

Ein auf einer optischen Messung beruhendes Analysesystem weist den Vorteil auf, dass eine sehr schnelle und einfache Messung der Änderung in der Analysezone 9a, 9b erfolgen kann. Diese berührungs- und kontaktlose Messung hat den Vorteil, dass eine räumliche Trennung des Testelements 3 und des Analysegeräts 2 stattfinden kann. Insbesondere bei der Verwendung von Lichtleitern, wie Lichtwellenleiter aus Polymer oder aus Glasfaser oder dergleichen, bietet die optische Messung einen großen Gestaltungsspielraum, insbesondere bei der Integration in bestehende kombinierte Stech- und Analysesysteme.

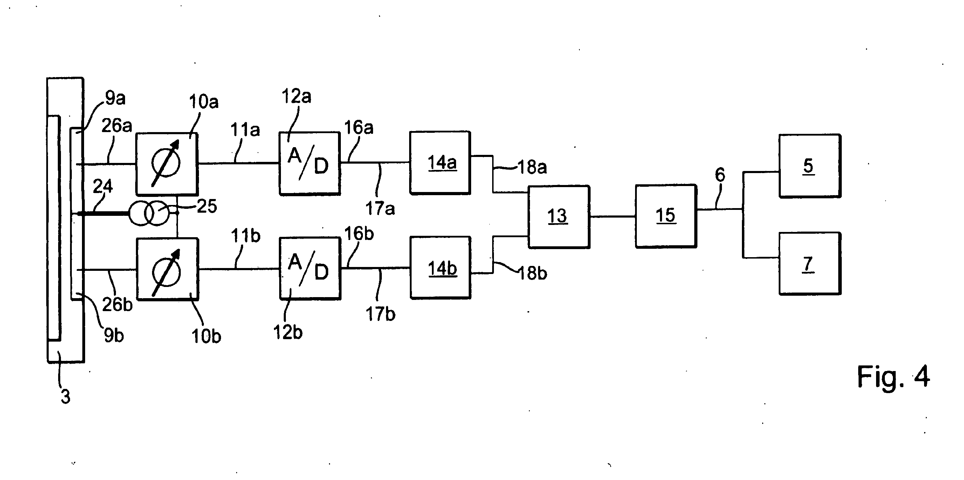

Figur 4 zeigt eine weitere bevorzugte Ausführungsform des erfindungsgemäßen Analysesystems nach Figur 2, dessen Messprinzip auf einer elektrochemischen Messung beruht. Ein elektrischer Leiter 24 verbindet eine elektrische Quelle 25, die beispielsweise eine Spannungsquelle oder eine Stromquelle sein kann, mit den beiden Analysezonen 9a und 9b. Zwei elektrische Leiter 26a, 26b kontaktieren die jeweiligen Analysezonen 9a, 9b mit den zugehörigen Analog-Messeinheiten 10a, 10b derart, dass ein Stromfluss durch den Leiter 24, die jeweilige Analysezone 9a, 9b und den elektrischen Leiter 26a, 26b (als Rückleiter) in der Analog-Messeinheit 10a bzw. 10b messbar ist. Selbstverständlich kann die Mess- und Auswerteeinheit 4 die im Stand der Technik bekannten Komponenten umfassen, so dass neben einer Polarisationsspannung auch in zeitlicher Reihenfolge unterschiedliche Wechselspannungen in der elektrischen Quelle 25 erzeugt werden können. Neben einer Strommessung kann dann auch zusätzlich die Bestimmung der Impedanz erfolgen, aus der weitere Informationen über die Probenflüssigkeit gewonnen werden können.

Alternativ können auch zwei getrennte Stromkreise aufgebaut werden, indem zwei elektrische Quellen vorgesehen sind, die jeweils mit einem elektrischen Leiter je eine der Analysezonen 9a, 9b kontaktieren.

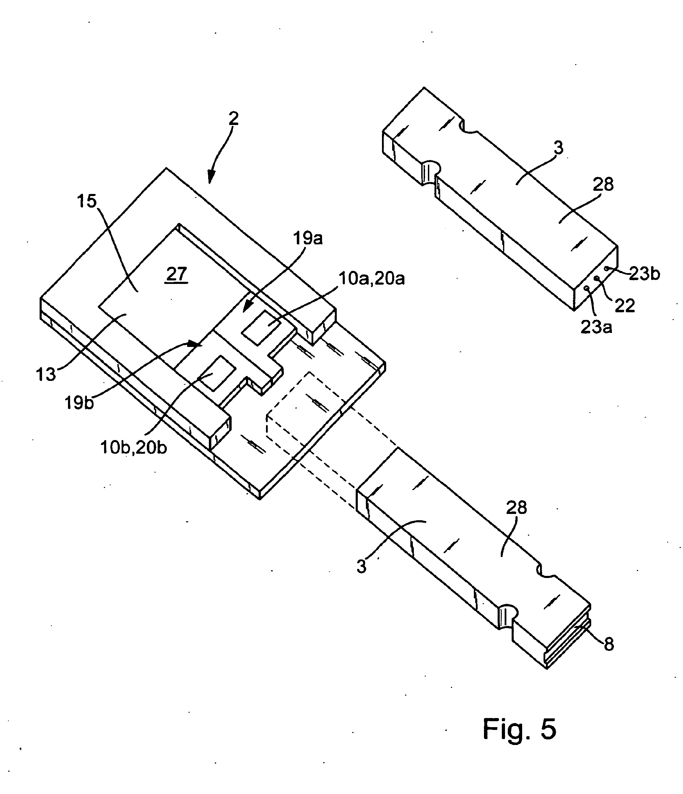

Figur 5 zeigt eine bevorzugte Ausführungsform eines Analysegerätes 2 mit zwei identisch aufgebauten Auswertekanälen 19a, 19b, die mit einer Elektronikeinheit 27 verbunden sind. Die Elektronikeinheit 27 umfasst die Vergleichereinheit 13 und die Verarbeitungsendeinheit 15. Das Analysegerät 2 verwendet ein optisches Messprinzip, so dass die Analog-Messeinheiten 10a, 10b je einen optischen Empfänger 20a, 20b umfassen.

Das Testelement 3 ist als Teststick 28 ausgebildet, an dessen einen Schmalseite die Probenaufgabezone 8 angeordnet ist. In dem Teststick 28 verlaufen drei Lichtleiter parallel, wobei der mittlere Lichtleiter 22 zur Übertragung von Licht von dem Analysegerät 2 zu den (hier nicht sichtbaren) Analysezonen 9a, 9b leitet, die unterhalb der Probenaufgabezone 8 angeordnet sind. Die beiden äußeren Lichtleiter 23a, 23b übertragen das von den Analysezonen 9a; 9b reflektierte Licht zurück zu den optischen Empfängern 20a, 20b des Analysegeräts 2.

Die Ausbildung des Testelements 3 als Teststick 28 weist den Vorteil auf, dass ein derartiger Teststick sehr gut handhabbar ist und bei der Benutzern auf eine hohe Akzeptanz stößt. Gleichzeitig ist eine deutliche Trennung von Probenaufgabezone 8 und der Mess- und Auswerteeinheit 4 realisiert. Dennoch arbeitet der Teststick 28 mit sehr kleinen Blutmengen im Bereich von 100 nl und darunter, da die Analysezonen 9a, 9b und die Probenaufgabezone 8 sehr kompakt und klein ausgebildet sind. Die Übertragungswege der Flüssigkeit sind sehr kurz. Durch die optische Übertragung mittels Licht kann die eigentliche Messtechnik entfernt von der Probenaufgabezone 8 in dem Gerät 2 integriert sein.

## Patentansprüche

1. Analysesystem zur Bestimmung eines Analyten einer Körperflüssigkeit, umfassend:
- ein Testelement (3) und
- ein Analysegerät (2) mit einer Mess- und Auswerteeinheit (4),
wobei
das Testelement (3)
- ein Reagenzsystem, dessen Reaktion mit dem Analyten zu einer für das gewünschte analytische Resultat charakteristischen nachweisbaren Änderung führt,
- eine Probenaufgabezone (8) und
- zwei unterhalb der Probenaufgabezone (8) angeordneten Analysezonen (9a, 9b) umfasst und,
wobei
die Mess- und Auswerteeinheit (4)
- zwei Analog-Messeinheiten (10a, 10b), in denen ein der Änderung in jeweils einer der Analysezonen (9a, 9b) korrespondierendes analoges Messsignal (11a, 11b) erzeugt wird,
- zwei Analog-Digital-Wandler (12a, 12b) zum Digitalisieren des analogen Messsignals (11a, 11b),
- eine Vergleichereinheit (13) zum Vergleichen von auf den digitalisierten Messsignalen (16a, 16b) basierenden Kontroll-Datenwerten (17a, 17b) und
- eine Verarbeitungsendeinheit (15), in der, wenn die ermittelte Abweichung zwischen den Kontroll-Datenwerten (17a, 17b) der digitalisierten Messsignale (16a, 16b) kleiner als ein vordefinierter Wert ist, eine Freigabe mindestens eines der Kontroll-Datenwerte (17a, 17b) und eine Weiterverarbeitung zu dem gesuchten analytischen Resultat (6) erfolgt, umfasst und,
wobei
eine der Analysezonen (9a, 9b), eine der Analog-Messeinheiten (10a, 10b) und einer der Analog-Digital-Wandler (12a, 12b) je einen Auswertekanal bilden, und
das Analysesystem (1) zwei redundante Auswertekanäle zum Erkennen eines Fehlers des Analysesystems (1) aufweist.

2. Analysesystem nach Anspruch 1, **dadurch gekennzeichnet, dass** die Kontroll-Datenwerte (17a, 17b) Konzentrationswerte (18a, 18b) sind und der vordefinierte Wert ein Referenzkonzentrationswert ist.

3. Analysesystem nach Anspruch 1 oder 2, **dadurch gekennzeichnet, dass** die Mess- und Auswerteeinheit (4) eine Auswerteeinheit (14) umfasst, in der aus den Kontroll-Datenwerten (17a, 17b) Konzentrationswerte (18a, 18b) mittels eines Auswertealgorithmus ermittelt werden und der Auswertealgorithmus eine Zuordnung zwischen den Kontroll-Datenwerten (17a, 17b) und Konzentrationswerten (18a, 18b) umfasst, wobei die Auswerteeinheit (14) zwischen der Vergleichereinheit (13) und der Verarbeitungsendeinheit (15) angeordnet ist.

4. Analysesystem nach Anspruch 1 oder 2, **dadurch gekennzeichnet, dass** die Mess- und Auswerteeinheit (4) zwei Auswerteeinheiten (14a, 14b) umfasst, in denen aus den digitalisierten Messsignalen (16a, 16b) Konzentrationswerte (18a, 18b) mittels je eines Auswertealgorithmus, der eine Zuordnung zwischen Werten des digitalisierten Messsignals (16a, 16b) und Konzentrationswerten (18a, 18b) umfasst, ermittelt werden, wobei die Auswerteeinheiten (14a, 14b) in der Verarbeitungskette vor der Vergleichereinheit (13) angeordnet sind.

5. Analysesystem nach Anspruch 3 oder 4, **dadurch gekennzeichnet, dass** die Zuordnung des Auswertealgorithmus durch eine Kalibration erfolgt, in dem zu Referenzkonzentrationswerten digitalisierte Referenzmesssignale erzeugt werden.

6. Analysesystem nach einem der vorhergehenden Ansprüche, **dadurch gekennzeichnet, dass**
- das Analysegerät (2) einen optischen Sender (21) zur Emission von Licht auf die Analysezonen (9a, 9b) aufweist, und
- die Mess- und Auswerteeinheit (4) zwei optische Empfänger (20a, 20b) zum Empfang von jeweils einer Analysezone (9a, 9b) reflektierten Lichts aufweist, und
- in den beiden Analog-Messeinheiten (10a, 10b) je ein analoges Messsignal (11a, 11b) erzeugt wird, das zu dem aus je einer der Analysezonen (9a, 9b) reflektierten Lichts proportional ist.

7. Analysesystem nach Anspruch 6, **dadurch gekennzeichnet, dass** ein Lichtleiter (22) zwischen dem optischen Sender (21) und den Analysezonen (9a, 9b) derart angeordnet ist, dass wenigstens eine der Analysezonen 9a, 9b) mit Licht bestrahlt wird, und
je zwischen einem optischen Empfänger (20a, 20b) und einer Analysezone (9a, 9b) ein Lichtleiter (23a, 23b) derart angeordnet ist, dass reflektiertes Licht von der Analysezone (9a, 9b) an den optischen Empfänger (20a, 20b) übertragen wird.

8. Analysesystem nach einem der Ansprüche 1 bis 5, **dadurch gekennzeichnet, dass** die Mess- und Auswerteeinheit (4) einen ersten elektrischen Leiter (24) umfasst, der mit wenigstens einer der Analysezonen (9a, 9b) kontaktiert ist, und einen zweiten elektrischen Leiter (26a, 26b), der mit einer Analysezone (9a, 9b) und einer Analog-Messeinheit (10a, 10b) derart kontaktiert ist, dass ein Stromfluss durch den ersten elektrischen Leiter (24), die wenigstens eine Analysezone (9a, 9b) und den zweiten elektrischen Leiter (26a, 26b) in der einen Analog-Messeinheit (10a, 10b) messbar ist.

9. Analysesystem nach einem der vorhergehenden Ansprüche, **dadurch gekennzeichnet, dass** zwischen der Probenaufgabezone (8) und den Analysezonen (9a, 9b) ein Kapillarkanal angeordnet ist, mittels dem die Körperflüssigkeit auf die Analysezonen (9a, 9b) übertragen wird.

10. Analysesystem nach einem der vorhergehenden Ansprüche, **dadurch gekennzeichnet, dass** die beiden Analysezonen (9a, 9b) voneinander verschiedene Analysevolumen aufweisen.

11. Analysesystem nach einem der vorhergehenden Ansprüche, **dadurch gekennzeichnet, dass** bei Unterschreiten des vordefinierten Wertes durch die Abweichung zwischen den beiden Kontroll-Datenwerten (17a, 17b) beide Kontroll-Datenwerte (17a, 17b) in der Verarbeitungsendeinheit (15) freigegeben werden und die Weiterverarbeitung zu dem gesuchten analytischen Resultat (6) auf Basis beider Kontroll-Datenwerte (17a, 17b) erfolgt.

12. Analysesystem nach Anspruch 11, **dadurch gekennzeichnet, dass** das analytische Resultat (6) aus dem Mittelwert der beiden Kontroll-Datenwerte (17a, 17b) ermittelt wird.

13. Analysesystem nach einem der Ansprüche 1 bis 11, **dadurch gekennzeichnet, dass** das analytische Resultat (6) aus dem kleineren oder dem größeren der beiden Kontroll-Datenwerte (17a, 17b) ermittelt wird.

14. Analysesystem nach einem der vorhergehenden Ansprüche, **dadurch gekennzeichnet, dass**, wenn die ermittelte Abweichung zwischen den Kontroll-Datenwerten (17a, 17b) der digitalisierten Messsignale (16a, 16b) größer als der vordefinierte Wert ist, keine Freigabe der Kontroll-Datenwerte (17a, 17b) erfolgt und ein Fehlersignal erzeugt wird.

15. Analysesystem nach einem der vorhergehenden Ansprüche, **dadurch gekennzeichnet, dass** das Analysegerät (2) eine Ausgabeeinheit (5) umfasst, an der das analytische Resultat (6) oder ein Fehlersignal ausgegeben wird.

## Claims

1. Analysis system for determining an analyte in a body fluid, comprising:
- a test element (3) and
- an analysis instrument (2) with a measurement and evaluation unit (4)
wherein
the test element (3) comprises:
- a reagent system, whose reaction with the analyte results in a detectable change which is characteristic for the desired analytical result,
- a sample application zone (8) and
- two analysis zones (9a, 9b), which are positioned below the sample application zone (8), and
wherein
the measurement and evaluation unit (4) comprises:
- two analog measuring units (10a, 10b), in each of which an analog measurement signal (11a, 11b) corresponding to the change in one of the analysis zones (9a, 9b) is generated,
- two analog-digital converters (12a, 12b) for digitizing the analog measurement signal (11a, 11b),
- a comparator unit (13) for comparing control data values (17a, 17b) based on the digitized measurement signals (16a, 16b), and
- a final processing unit (15), in which, if a determined deviation between the control data values (17a, 17b) of the digitized measurement signals (16a, 16b) is less than a predefined value, at least one of the control data values (17a, 17b) is allowed to pass for further processing into the desired analytical result (6) and
wherein
one of the analysis zones (9a, 9b), one of the analog measuring units (10a, 10b) and one of the analog-digital converters (12a, 12b) form an evaluation channel and the analysis system (1) has two redundant evaluation channels for detecting an error of the analysis system (1).

2. Analysis system according to claim 1, **characterized in that** the control data values (17a, 17b) are concentration values (18a, 18b) and the predefined value is a reference concentration value.

3. Analysis system according to claim 1 or 2, **characterized in that** the measurement and evaluation unit (4) comprises an evaluation unit (14) in which concentration values (18a, 18b) are determined from the control data values (17a, 17b) using an evaluation algorithm and the evaluation algorithm comprises an assignment between the control data values (17a, 17b) and concentration values (18a, 18b), the evaluation unit (14) being positioned between the comparator unit (13) and the final processing unit (15).

4. Analysis system according to claim 1 or 2, **characterized in that** the measurement and evaluation unit (4) comprises two evaluation units (14a, 14b) in which concentration values (18a, 18b) are determined from the digitized measurement signals (16a, 16b) each using an evaluation algorithm, which comprises an assignment between values of the digitized measured signals (16a, 16b) and concentration values (18a, 18b), the evaluation units (14a, 14b) being positioned in the processing chain before the comparator unit (13).

5. Analysis system according to claim 3 or 4, **characterized in that** the assignment of the evaluation algorithm is performed by a calibration wherein digitized reference measurement signals are generated for reference concentration values.

6. Analysis system according to any one of the preceding claims, **characterized in that**
- the analysis instrument (2) has an optical transmitter (21) for emitting light onto the analysis zones (9a, 9b), and
- the measurement and evaluation unit (4) has two optical receivers (20a, 20b) each for receiving light from one analysis zone (9a, 9b), and
- an analog measurement signal (11a, 11b) is generated in each of the two analog measuring units (10a, 10b), which analog signals (11a, 11b) are proportional to the light reflected from each of the analysis zones (9a, 9b).

7. Analysis system according to claim 6, **characterized in that** an optical fiber (22) is positioned between the optical transmitter (21) and an analysis zone (9a, 9b) in such a manner that at least one of the analysis zones (9a, 9b) is irradiated with light, and
an optical fiber (23a, 23b) is positioned between each optical receiver (20a, 20b) and each analysis zone (9a, 9b) in such a manner that reflected light from the analysis zone (9a, 9b) is transmitted to the optical receivers (20a, 20b).

8. Analysis system according to any one of claims 1 to 5, **characterized in that** the measurement and evaluation unit (4) comprises a first electrical conductor (24) which is contacted with at least one of the analysis zones (9a, 9b), and a second electrical conductor (26a, 26b), which is contacted with an analysis zone (9a, 9b) and an analog measuring unit (10a, 10b) in such a manner that a current flow through the first electrical conductor (24), the at least one analysis zone (9a, 9b), and the second electrical conductor (26a, 26b) is measurable by the one analog measuring unit (10a, 10b).

9. Analysis system according to any one of the preceding claims, **characterized in that** a capillary channel is positioned between the sample application zone (8) and the analysis zones (9a, 9b), whereby the body fluid is transferred onto the analysis zones (9a, 9b).

10. Analysis system according to any one of the preceding claims, **characterized in that** the two analysis zones (9a, 9b) have analysis volumes different from one another.

11. Analysis system according to any one of the preceding claims, **characterized in that**, if the deviation between the two control data values (17a, 17b) is less than the predefined value, both control data values (17a, 17b) are enabled in the final processing unit (15) and **in that** the further processing into the desired analytical result (6) is performed on the basis of both control data values (17a, 17b).

12. Analysis system according to claim 11, **characterized in that** the analytical result (6) is determined from the mean value of the two control data values (17a, 17b).

13. Analysis system according to any one of claims 1 to 11, **characterized in that** the analytical result (6) is determined from the lower or higher of the two control data values (17a, 17b).

14. Analysis system according to any one of the preceding claims, **characterized in that**, if the determined deviation between the control data values (17a, 17b) of the digitized measurement signals (16a, 16b) is more than the predefined value, the control data values (17a, 17b) are not allowed to pass and an error signal is generated.

15. Analysis system according to any one of the preceding claims, **characterized in that** the analysis instrument (2) comprises an output unit (5), at which the analytical result (6) or an error signal is outputted.

## Revendications

1. Système d'analyse destiné à déterminer un analyte d'un liquide corporel, comprenant :
- un élément de test (3) et
- un appareil d'analyse (2) avec une unité de mesure et d'évaluation (4),
dans lequel ledit élément de test (3) comprend
- un système de réactifs dont la réaction avec l'analyte conduit à une modification décelable caractéristique du résultat analytique souhaité,
- une zone de réception d'échantillons (8) et
- deux zones d'analyse (9a, 9b) disposées au-dessous de la zone de réception d'échantillons (8), et
dans lequel ladite unité de mesure et d'évaluation (4) comprend
- deux unités de mesure analogiques (10a, 10b) dans lesquelles est généré un signal de mesure analogique (11a, 11b) correspondant à la modification dans l'une des zones d'analyse (9a, 9b),
- deux convertisseurs analogiques-numériques (12a, 12b) destinés à numériser le signal de mesure analogique (11a, 11 b),
- une unité de comparaison (13) destinée à comparer des valeurs de données de contrôle (17a, 17b) basées sur les signaux de mesure numérisés (16a, 16b), et
- une unité terminale de traitement (15) dans laquelle, lorsque l'écart déterminé entre les valeurs de données de contrôle (17a, 17b) des signaux de mesure numérisés (16a, 16b) est inférieur à une valeur prédéfinie, ont lieu une validation d'au moins l'une des valeurs de données de contrôle (17a, 17b) et un traitement ultérieur conduisant au résultat analytique recherché (6), et
dans lequel l'une des zones d'analyse (9a, 9b), l'une des unités de mesure analogiques (10a, 10b) et l'un des convertisseurs analogiques-numériques (12a, 12b) forment un canal d'évaluation, et
le système d'analyse (1) présente deux canaux d'évaluation redondante destinés à détecter une erreur du système d'analyse (1).

2. Système d'analyse selon la revendication 1, **caractérisé en ce que** les valeurs de données de contrôle (17a, 17b) sont des valeurs de concentration (18a, 18b) et la valeur prédéfinie est une valeur de concentration de référence.

3. Système d'analyse selon la revendication 1 ou 2, **caractérisé en ce que** l'unité de mesure et d'évaluation (4) comprend une unité d'évaluation (14) dans laquelle sont déterminées à partir des valeurs de données de contrôle (17a, 17b) des valeurs de concentration (18a, 18b) au moyen d'un algorithme d'évaluation et l'algorithme d'évaluation comprend une mise en correspondance entre les valeurs de données de contrôle (17a, 17b) et les valeurs de concentration (18a, 18b), ladite unité d'évaluation (14) étant disposée entre l'unité de comparaison (13) et l'unité terminale de traitement (15)

4. Système d'analyse selon la revendication 1 ou 2, **caractérisé en ce que** l'unité de mesure et d'évaluation (4) comprend deux unités d'évaluation (14a, 14b) dans lesquelles sont déterminées à partir des signaux de mesure numérisés (16a, 16b) des valeurs de concentration (18a, 18b) au moyen d'un algorithme d'évaluation comprenant une mise en correspondance entre les valeurs du signal de mesure numérisé (16a, 16b) et les valeurs de concentration (18a, 18b), lesdites unités d'évaluation (14a, 14b) étant disposées dans la chaîne de traitement en amont de l'unité de comparaison (13).

5. Système d'analyse selon la revendication 3 ou 4, **caractérisé en ce que** la mise en correspondance de l'algorithme d'évaluation s'effectue par calibration, en générant pour des valeurs de concentration de référence des signaux de mesure de référence numérisés.

6. Système d'analyse selon l'une des revendications précédentes, **caractérisé en ce que**
- l'appareil d'analyse (2) présente un émetteur optique (21) destiné à émettre de la lumière sur les zones d'analyse (9a, 9b), et
- l'unité de mesure et d'évaluation (4) présente deux récepteurs optiques (20a, 20b) destinés à recevoir la lumière réfléchie par chacune des zones d'analyse (9a, 9b), et
- un signal de mesure analogique (11a, 11b) est généré dans les deux unités de mesure analogiques (10a, 10b), lequel est proportionnel à la lumière réfléchie à partir de chacune des zones d'analyse (9a, 9b).

7. Système d'analyse selon la revendication 6, **caractérisé en ce qu'**un guide de lumière (22) est disposé entre l'émetteur optique (21) et les zones d'analyse (9a, 9b), de telle sorte qu'au moins l'une des zones d'analyse (9a, 9b) est exposée aux rayons de lumière, et
un guide de lumière (23a, 23b) est disposé à chaque fois entre un récepteur optique (20a, 20b) et une zone d'analyse (9a, 9b), de telle sorte que la lumière réfléchie est transmise de la zone d'analyse (9a, 9b) au récepteur optique (20a, 20b).

8. Système d'analyse selon l'une des revendications 1 à 5, **caractérisé en ce que** l'unité de mesure et d'évaluation (4) comprend un premier conducteur électrique (24) en contact avec au moins l'une des zones d'analyse (9a, 9b), et un deuxième conducteur électrique (26a, 26b) en contact avec une zone d'analyse (9a, 9b) et une unité de mesure analogique (10a, 10b), de telle sorte qu'un flux de courant traversant ledit premier conducteur électrique (24), ladite au moins une zone d'analyse (9a, 9b) et ledit deuxième conducteur électrique (26a, 26b) peut être mesuré dans l'unité de mesure analogique (10a, 10b).

9. Système d'analyse selon l'une des revendications précédentes, **caractérisé en ce qu'**un canal capillaire est disposé entre la zone de réception d'échantillons (8) et les zones d'analyse (9a, 9b), au moyen duquel le liquide corporel est transférée sur les zones d'analyse (9a, 9b).

10. Système d'analyse selon l'une des revendications précédentes, **caractérisé en ce que** les deux zones d'analyse (9a, 9b) ont des volumes d'analyse différents.

11. Système d'analyse selon l'une des revendications précédentes, **caractérisé en ce que**, lorsque l'écart entre les deux valeurs de données de contrôle (17a, 17b) est inférieur à la valeur prédéfinie, lesdites deux valeurs de données de contrôle (17a, 17b) sont validées dans l'unité terminale de traitement (15) et le traitement ultérieur conduisant au résultat analytique recherché (6) a lieu sur la base desdites deux valeurs de données de contrôle (17a, 17b).

12. Système d'analyse selon la revendication 11, **caractérisé en ce que** le résultat analytique (6) est déterminé à partir de la moyenne des deux valeurs de données de contrôle (17a, 17b).

13. Système d'analyse selon l'une des revendications précédentes, **caractérisé en ce que** le résultat analytique (6) est déterminé à partir de la valeur inférieure ou supérieure des deux valeurs de données de contrôle (17a, 17b).

14. Système d'analyse selon l'une des revendications précédentes, **caractérisé en ce que**, lorsque l'écart déterminé entre les valeurs de données de contrôle (17a, 17b) des signaux de mesure numérisés (16a, 16b) est supérieur à la valeur prédéfinie, il n'y pas validation desdites valeurs de données de contrôle (17a, 17b) et un signal d'erreur est généré.

15. Système d'analyse selon l'une des revendications précédentes, **caractérisé en ce que** l'appareil d'analyse (2) comprend une unité de sortie (5) sur laquelle est délivré le résultat analytique (6) ou un signal d'erreur.
